# EUROPEAN PATENT APPLICATION

(11) **EP 1 712 546 A1**
(43) Date of publication of application: **18.10.2006**
(21) Application number: 06007914.2
(22) Date of filing: 13.04.2006
(51) Int. Cl.: C07C 243/38

(54) **Hydroxynaphthalenedicarboxylic acid hydrazide and derivatives thereof as well as process for preparing them**

(30) Priority: 14.04.2005 JP 2005116809
(71) Applicant: Kabushiki Kaisha Ueno Seiyaku Oyo Kenkyujo, Osaka-shi, Osaka-fu (JP)
(72) Inventor: Wakamori, Hiroyuki, Tanba-shi Hyogo-ken (JP)
(74) Representative: Albrecht, Thomas

(57) **Abstract**

The - present invention provides a hydroxynaphthalenedicarboxylic acid hydrazide or a derivative thereof represented by formula (1): 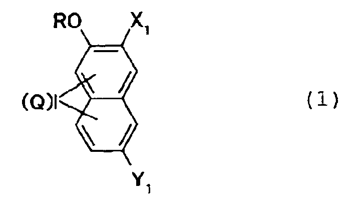 wherein X₁ is a group selected from the group consisting of carboxyl group, a group represented by formula (2) and a group represented by formula (3):

-CO-NH-Z (2)

-CO-NHNH₂ (3)

Y₁ is a group selected from the group consisting of carboxyl group, carbamoyl group, a group represented by formula (2), a group represented by formula (3) and a group represented by formula (4):

-CO-O-A (4)

provided that at least one of X₁ and Y₁ is a group represented by formula (3).

## Description

### Technical Field

The present invention relates to a novel hydroxynaphthalenedicarboxylic acid hydrazide and derivatives thereof as well as a process for preparing the same.

### Background art

Hydroxynaphthalenecarboxylic acids such as 2-hydroxy-3-naphthoic acid, 2-hydroxy-6-naphthoic acid and 2-hydroxynaphthalene-3,6-dicarboxylic acid are widely used for synthesis of various products such as organic coloring matters and synthetic resins.

Some of 2-hydroxy-3-naphthoic acid compounds and their hydrazide derivatives have been used as synthetic raw materials of coupler components of azo compounds which are used as charging materials of electrophotographic photo conductors (see Japanese patent Application Laid Open Number Hei 6-95403), as rubber additives for tires or synthetic intermediates thereof (see Japanese patent Application Laid Open Numbers Hei 4-136048 and Hei 11-292834) and as curing agents or hardening accelerators for epoxy resins (see Japanese patent Application Laid Open Number Hei 9-67466). These cited references are herein incorporated by reference.

To -the date, neither 2-hydroxynaphthalene-3,6-dicarboxylic acid hydrazide nor its derivative is known. 2-Hydroxynaphthalene-3,6-dicarboxylic acid hydrazide or its derivative may be useful as a coupler component of a novel azo compound and as a synthetic raw material of a novel rubber additive.

### Disclosure of the invention

An object of the present invention is to provide a novel hydroxynaphthalenedicarboxylic acid hydrazide and a derivative thereof as well as a process for preparing the same.

The present invention provides a hydroxynaphthalenedicarboxylic acid hydrazide or a derivative thereof represented by formula (1): 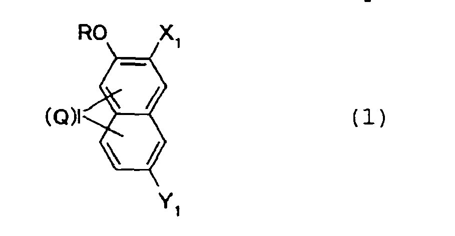 wherein X₁ is a group selected from the group consisting of carboxyl group, a group represented by formula (2) and a group represented by formula (3):

-CO-NH-Z (2)

-CO-NHNH₂ (3)

wherein Z is a group selected from the group consisting of an optionally branched, optionally substituted, saturated or unsaturated aliphatic group having 1-20 carbon atoms, an optionally substituted aromatic group and an optionally substituted heterocyclic group having conjugated double bonds;
Y₁ is a group selected from the group consisting of carboxyl group, carbamoyl group, a group represented by formula (2), a group represented by formula (3) and a group represented by formula (4):

-CO-O-A (4)

wherein A is alkyl group having 1-6 carbon atoms;
provided that at least one of X₁ and Y₁ in formula (1) is a group represented by formula (3);
R is selected from the group consisting of hydrogen atom, an optionally branched alkyl group having 1-20 carbon atoms which may be optionally substituted with hydroxyl group and/or halogen atom, and aralkyl group having 7-11 carbon atoms;
Q is selected from the group consisting of alkyl group having 1-6 carbon atoms, alkoxy group having 1-6 carbon atoms, halogen atom, nitro group and hydroxyl group;
1 represents an integer of 0-5;
provided that when 1 is from 2 to 5, the Qs may be the same or different.

In the specification and claims attached herewith, the term "aromatic group" represents a 6-membered monocyclic aromatic group or a condensed ring group consisting of up to 4 of the condensed aromatic rings.

"Heterocyclic group having conjugated double bonds" represents a 5- or 6-membered monocyclic group or condensed ring group having at least one heteroatom selected from N, S and O and conjugated double bonds. When it constitutes a condensed ring group, said group may have up to 6 rings.

In a hydroxynaphthalenedicarboxylic acid hydrazide or a derivative thereof of the present invention represented by formula (1) wherein at least one of X₁ and Y₁ is represented by formula (2), examples of the X₁ and Y₁ include alkylaminocarbonyl group, naphthylaminocarbonyl group, phenylaminocarbonyl group and the like.

In the present compounds wherein Z in formula (2) is an optionally branched, optionally substituted, saturated or unsaturated aliphatic group having 1-20 carbon atoms, examples of the Z include methyl group, ethyl group, n-propyl group, isopropyl group, n-butyl group, n-hexyl group, n-octyl group, n-nonyl group, n-decyl group, n-dodecyl group, n-hexadecyl group, n-octadecyl group, 2-chloroethyl group, vinyl group, allyl group and the like.

In the present compounds wherein Z in formula (2) is an optionally substituted aromatic group, examples of the Z include benzene ring, naphthalene ring, anthraquinone ring and the like. In the present compounds of which Z in formula (2) is an optionally substituted heterocyclic group having conjugated double bonds, examples of the Z include thiophene, furan, pyrrole, imidazole, pyrazole, isothiazole, isoxazole, pyridine, pyrazine, pyrimidine, pyridazine, triazole, tetrazole, indole, 1H-indazole, purine, 4H-quinolizine, isoquinoline, quinoline, phthalazine, naphthyridine, quinoxaline, quinazoline, cinnoline, pteridine, benzofuran and the like.

Examples of substituents on Z include halogen atom, halogenated C₁₋₆ alkyl, nitro group, C₁₋₆ alkyl group, C₁₋₆ alkoxy group (such as methoxy group), cyano group, phenoxy group, phenylamino group, benzoylamino group, penylcarbamoyl group, alkylaminosulfonyl group and C₂₋₆ alkenyl group optionally having aryl group and the like. When the substituent on Z contains aromatic ring group, said ring may have one or more further substituents such as halogen atom, C₁₋₆ alkyl group, C₁₋₆ alkoxy group, phenyl group, cyano group and the like.

In the compound of the present invention represented by formula (1) wherein Y₁ is a group represented by formula (4), examples of the Y₁ include methoxycarbonyl group, ethoxycarbonyl group, n-butoxycarbonyl group and the like.

In a hydroxynaphthalenedicarboxylic acid hydrazide or a derivative thereof represented by formula (1) of the present invention, R is selected from the group consisting of hydrogen atom, an optionally branched alkyl group having 1-20 carbon atoms which may be optionally substituted with hydroxyl group and/or halogen atom and aralkyl group having 7-11 carbon atoms.

In the compound of the present invention wherein R is an optionally branched alkyl group having 1-20 carbon atoms which may be optionally substituted with hydroxyl group and/or halogen atom, examples of the R include ethyl group, n-propyl group, isopropyl group, n-butyl group, isobutyl group, t-butyl group, n-pentyl group, n-hexyl group, n-octyl group, n-nonyl group, n-dodecyl group, n-hexadecyl group, n-octadecyl group, 2-hydroxyethyl group, 2-chloroethyl group, 4-hydroxybutyl group and the like. In the compound of the present invention of which R is an aralkyl group having 7-11 carbon atoms, examples of the R include benzyl group, phenethyl group, phenylpropyl group, 1-naphthylmethyl group, 2-naphthylmethyl group and the like.

In a hydroxynaphthalenedicarboxylic acid hydrazide or a derivative thereof of the present invention represented by formula (1), Q represents substituent(s) on the naphthalene ring. Examples of the Q include C₁₋₆ alkyl group, C₁₋₆ alkoxy group, halogen atom, nitro group and hydroxyl group. In formula (1) of the present invention, 1 represents an integer of 0-5, which is the number of substituents on the naphthalene ring. When 1 represents 2-5, i.e., when the naphthalene ring is substituted with two or more substituents, the Qs may be the same or different.

In the synthesis of a hydroxynaphthalenedicarboxylic acid hydrazide or a derivative thereof represented by formula (1) of the present invention, the starting material, which is a derivative of hydroxynaphthalenedicarboxylic acid represented by formula (5) may be prepared by any known method: 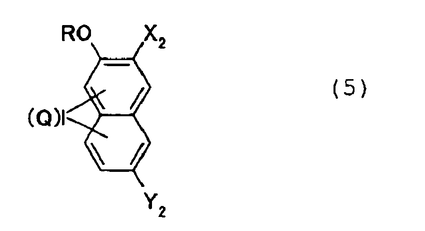 wherein X₂ and Y₂ are independently selected from the group consisting of a group represented by formula (2), a group represented by formula (3), a group represented by formula (4), carbamoyl group and carboxyl group;
provided that at least one of X₂ and Y₂ is a group represented by formula (4) or carbamoyl group;
R, Q and 1 are the same as defined above regarding to formula (1).

For example, a compound of formula (5) wherein both of X₂ and Y₂ are formula (4):

-CO-O-A (4)

wherein A is alkyl group having 1-6 carbon atoms or carbamoyl groups may be prepared according to scheme 1 below.

Specifically, 2-hydroxynaphthalene-3,6-dicarboxylic acid or a derivative thereof represented by formula (6) is reacted with thionyl chloride in solvent such as tetrahydrofuran to give an acid chloride represented by formula (7), which is reacted with a C₁₋₆ alcohol represented by A-OH to give an ester derivative of the hydroxynaphthalenedicarboxylic acid represented by formula (8). When the acid chloride represented by formula (7) is reacted with ammonia, a derivative of the hydroxynaphthalenedicarboxylic acid having carbamoyl group represented by formula (9) may be obtained. 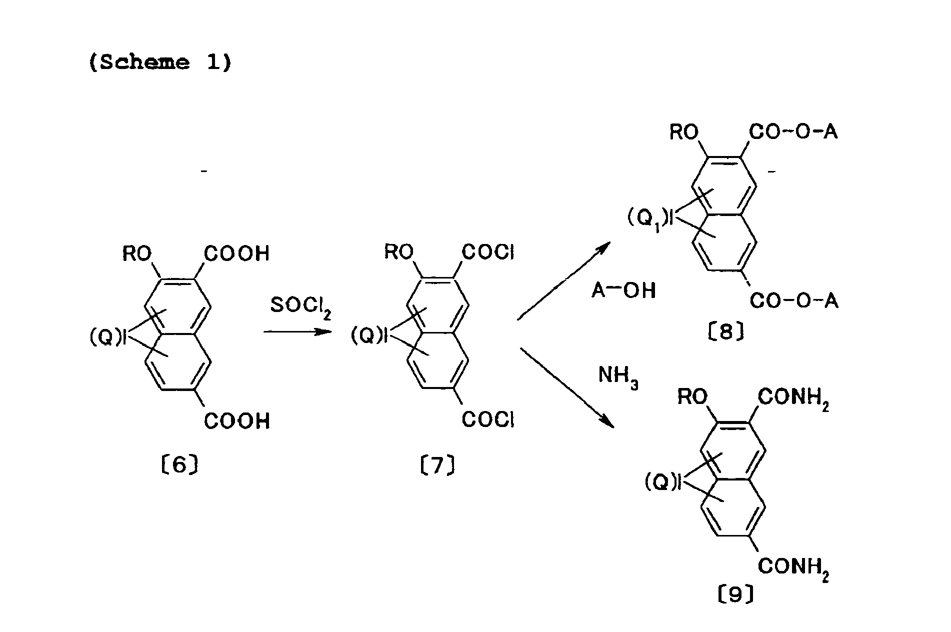 wherein R, Q and 1 in formulae (6)-(9) are the same as defined above with regard to formula (1); A-OH represents C₁₋₆ alcohol.

A compound represented by formula (8) may also be prepared by reacting a compound represented by formula (6) with A-OH, i.e., C₁₋₆ alcohol in the presence of acid such as sulfuric acid, para-toluenesulfonic acid and the like.

In case where a compound represented by formula (6) is 2-hydroxynaphthalene-3,6-dicarboxylic acid, the compound may be prepared according to the method disclosed in WO98/17621 (the cited reference is herein incorporated by reference). 2-Hydroxynaphthalene-3,6-dicarboxylic acid derivative represented by formula (6) may be prepared according to the conventional method which comprises alkylating or aralkylating the 2-hydroxyl group of 2-hydroxynaphthalene-3,6-dicarboxylic acid and introducing one or more substituent on the naphthalene ring.

In the present invention, a derivative of a hydroxynaphthalenedicarboxylic acid represented by formula (5) wherein one of X₂ and Y₂ is carboxyl group and the other is a group represented by formula (4) or carbamoyl group may be prepared according to any one of schemes 2-1 to 2-4 to give a compound represented by formula (13), (16), (19) or (22).

In the present invention, a derivative of a hydroxynaphthalenedicarboxylic acid represented by formula (5) wherein one of X₂ and Y₂ is a group represented by formula (4) and the other is carbamoyl group may be prepared according to scheme 2-3 or 2-4 to give a compound represented by formula (18) or (21).

Processes for preparing compounds represented by formulae (13), (16), (19), (22), (18) and (21) are further described herein below, but the processes do not limit the scope of the present invention.

### 1) Formula (13) (scheme 2-1)

Substituent Q is, if desired, introduced on the naphthalene ring of 2-hydroxynaphthalene-3,6-dicarboxylic acid according to the conventional method. Then, the carboxyl group on 3-position of the naphthalene ring is selectively esterified in solvent such as N,N-dimethylformamide. Alternatively, the carboxyl-group on 3-position of 2-hydroxynaphthalene-3,6-dicarboxylic acid is selectively esterified as described above and then substituent Q is introduced on the naphthalene ring to give a compound represented by formula (12). The reason why the selective esterification of carboxyl group on 3-position is achieved is that the influence of hydroxyl group on 2-position of the naphthalene ring causes the higher reactivity of 3-position than that of 6-position.

Thereafter, if desired, the hydroxyl group of 2-position is alkylated or aralkylated according to the conventional method to give a compound represented by formula (5) wherein X₂ is a group represented by formula (4) and Y₂ is carboxyl group (formula (13)).

### 2) Formula (16) (scheme 2-2)

2-Hydroxy-3,6-dicarboxylic acid which may have substituent Q (formula (10)) is esterified by, for example, reacting the compound of formula (10) with C₁₋₆ alcohol represented as A-OH in the presence of acid to give a compound represented by formula (14).

Thereafter, the ester group on 3-position of the compound of formula (14) is selectively hydrolyzed by weakly basic compound such as sodium bicarbonate and then acid is added to the reaction to give a compound represented by formula (15). The selective hydrolysis of the ester group of 3-position is achieved because of the same reason as described above.

Then, if desired, the hydroxyl group of 2-position is alkylated or aralkylated according to the conventional method to give a compound represented by formula (5) wherein X₂ is carboxyl group and Y₂ is a group represented by formula (4) (formula (16)).

### 3) Formulae (18), (19), (21) and (22) (schemes 2-3 and 2-4)

A compound obtained by scheme 2-1 (formula (13)) or that obtained by scheme 2-2 (formula (16)) is reacted with thionyl chloride in solvent such as tetrahydrofuran to give an acid chloride (formula (17) or formula (20)), which is reacted with ammonia to give a compound represented by formula (5) wherein one of X₂ and Y₂ is carbamoyl group and the other is a group represented by formula (4) (formula (18) or (21)). Then, the resulting compound represented by formula (5) (formula (18) or (21)) is hydrolyzed by basic compound such as sodium hydroxide in aqueous solvent to give a compound represented by formula (5) wherein one of X₂ and Y₂ is carbamoyl group and the other is carboxyl group (formula (19) or formula (22)).

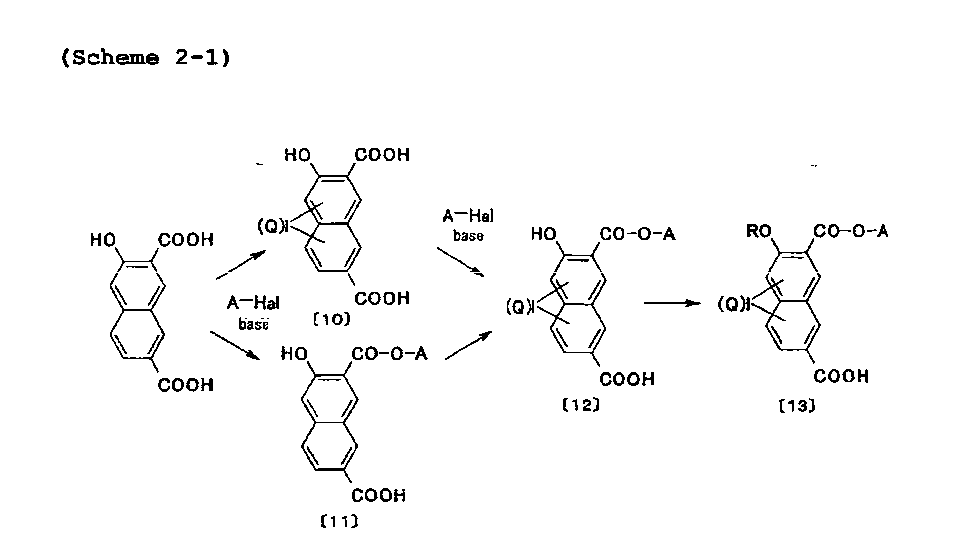

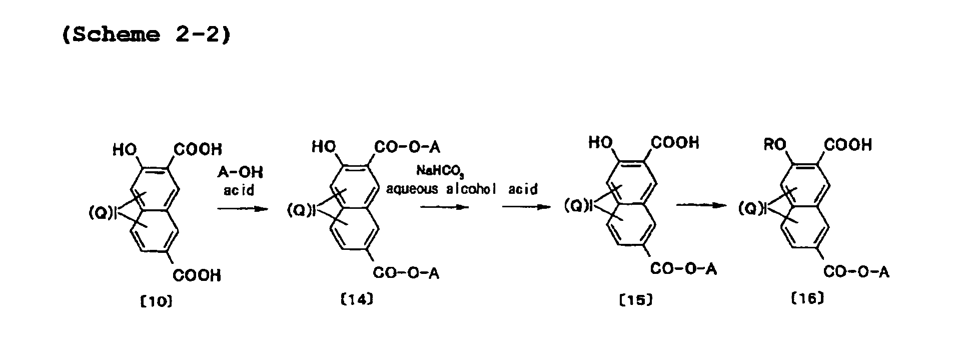

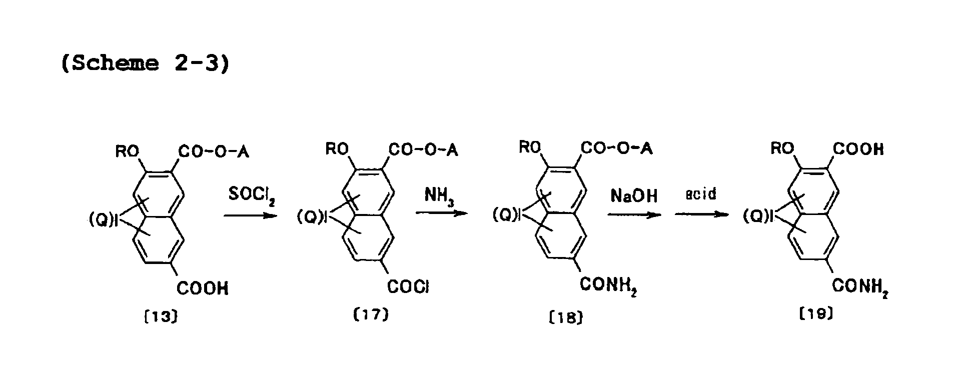

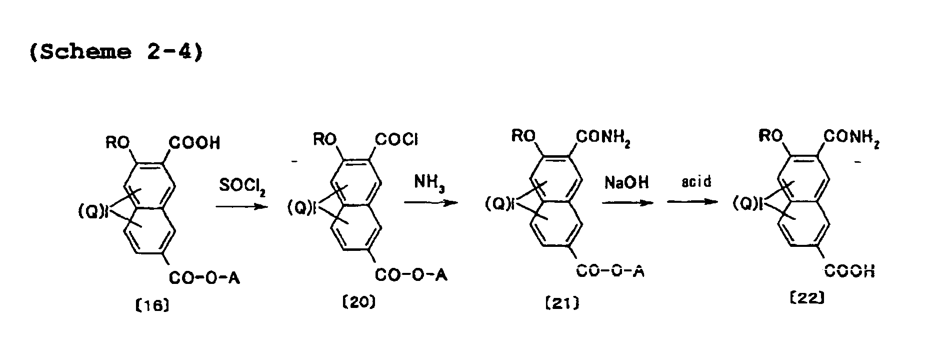

In formulae (10)-(22), R, Q and 1 are the same as defined with regard to formula (1). A-OH represents C₁₋₆ alcohol and A-Hal represents C₁₋₆ halogenated alkyl.

A compound represented by formula (5) wherein one of X₂ and Y₂ is a group represented by formula (2) may be obtained by amidation of a carboxyl group of a compound represented by formula (13), (16), (19) or (22) with an amine represented by Z-NH₂ according to the conventional method.

The hydroxynaphthalenedicarboxylic acid derivative represented by formula (5) obtained as above is subjected to the reaction with a hydrazine compound which is selected from the group consisting of hydrazine monohydrate, hydrazine sulfate, dihydrazine sulfate, hydrazine monohydrochloride, hydrazine dihydrochloride and hydrazine hydrobromide to give a hydroxynaphthalenedicarboxylic acid hydrazide or a derivative thereof.

Among the above hydrazine compounds, hydrazine monohydrate is preferably used because its side-product is water, which may be easily removed.

The amount of a hydrazine compound used for the reaction may preferably be 1.2-5.0 moles, and more preferably 2.0-2.5 moles per one mole of the total amount of the group represented by formula (4) and carbamoyl group which are present in a hydroxynaphthalenedicarboxylic acid derivative represented by formula (5).

The temperature of the reaction of the hydroxynaphthalenedicarboxylic acid derivative represented by formula (5) and a hydrazine compound may preferably be 20-110°C and more preferably 80-100°C for high reaction rate and less side product.

Solvents used for the hydrazidation reaction are not limited as long as they are inactive to the reaction. Examples of such solvents include alcohols such as methanol, ethanol, n-propanol, n-butanol and 2-ethylhexyl alcohol.

The hydrazidation reaction may be carried out until no less than 80 mole %, preferably no less than 90 mole % and more preferably no less than 95 mole % of the hydroxynaphthalenedicarboxylic acid derivative represented by formula (5), the starting material, is converted to the hydrazide derivative. The conversion rate can be confirmed with HPLC and the like.

The reaction time may vary depending on the reaction temperature and the solvent used, and in general, it may be 5-100 hours.

The hydrazidation reaction may be conducted by any manner known to the art and may be by means of batchwise reaction or continuous reaction.

After the hydrazidation reaction is completed, the hydroxynaphthalenedicarboxylic acid hydrazide or derivative thereof represented by formula (1) is precipitated, for example, by cooling, concentrating or adding poor solvent such as water. Thereafter, the precipitate is separated from the reaction mixture by any known means such as centrifugation and filter press and then dried.

The resulting hydroxynaphthalenedicarboxylic acid hydrazide or derivative thereof represented by formula (1) may be purified by recrystallization or washing it with organic solvent and/or water as desired. When one or more substituent selected from the group consisting of C₁₋₆ alkyl group, C₁₋₆ alkoxy group, halogen atom, nitro group and hydroxyl group can be introduced on the naphthalene ring. The substituents may be introduced according to a conventional method.

The hydroxynaphthalenedicarboxylic acid hydrazide or derivative thereof obtained by the process of the present invention may be suitably used as a coupler component of azo compound, a rubber additive for tires and an curing agent/hardening accelerator for epoxy resins or as a synthetic raw material for these products.

### Brief Description of the Drawings

Figure 1 is an infrared absorption spectrum of the compound of formula (I) obtained in Example 1.
Figure 2 is an infrared absorption spectrum of the compound of formula (II) obtained in Example 2.
Figure 3 is an infrared absorption spectrum of the compound of formula (III) obtained in Example 3.
Figure 4 is an infrared absorption spectrum of the compound of formula (IV) obtained in Example 5.
Figure 5 is an infrared absorption spectrum of the compound of formula (V) obtained in Example 6.

The present invention is further described in reference to the following examples. The examples are intended to illustrate the invention and not to be construed to limit the scope of the invention.

### Example 1

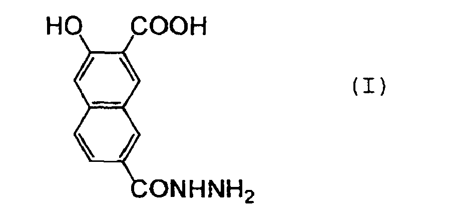

36.0 g (125 mmol) of 2-Hydroxy-3-hydroxycarbonyl-6-n-butoxycarbonylnaphthalene obtained by a known method was suspended in 100 g of n-butanol and to this mixture, 17.3 g (275 mmol) of hydrazine monohydrate was added dropwise at room temperature.

The reaction mixture was heated from room temperature to 100°C over one hour and kept at this temperature with stirring. At this temperature, the hydrazidation reaction was carried out for 72 hours and the resulting reaction mixture was analyzed by HPLC, which confirmed that the conversion rate of 2-hydroxy-3-hydroxycarbonyl-6-n-butoxycarbonylnaphthalene was no less than 95 mol %. The reaction mixture was cooled to room temperature and the hydrazidation reaction was completed.

The precipitate was separated from the reaction mixture by means of suction filtration to give the crude crystal of 2-hydroxy-3-hydroxycarbonyl-6-naphthoic acid hydrazide. The resulting crude crystal was washed by suspending it in 80 g of cold methanol, filtrated and dried to give 28.0 g (113.7 mmol) of the white crystal of 2-hydroxy-3-hydroxycarbonyl-6-naphthoic acid hydrazide (formula (I)).

Mass spectrum: m/z (-) 245, m/z (+) 247 (MW 246.2)

Decomposition point-:376°C

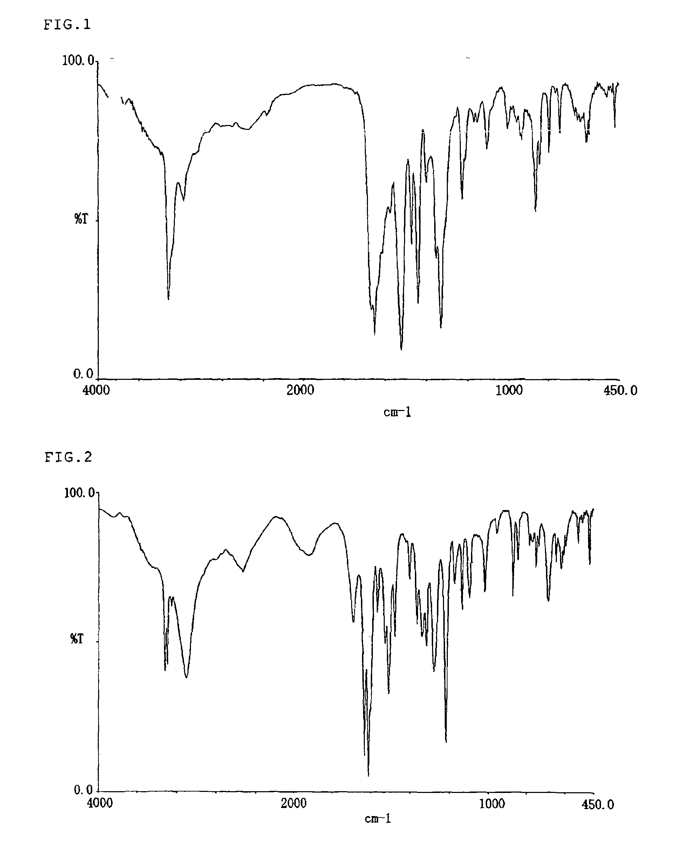

The infrared absorption spectrum (KBr method) of the resulting 2-hydroxy-3-hydroxycarbonyl-6-naphthoic acid hydrazide is shown in Figure 1.

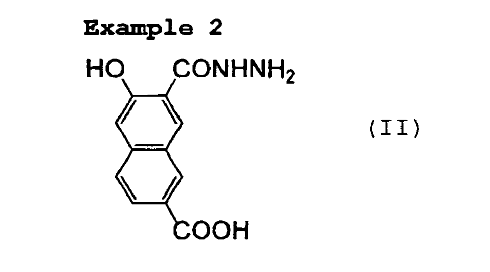

According to the same manner as described in Example 1 with the exception that 30.7 g of 2-hydroxy-6-hydroxycarbonyl-3-methoxycarbonylnaphthalene was used in stead of 2-hydroxy-3-hydroxycarbonyl-6-n-butoxycarbonylnaphthalene, 25.2 g (102.5 mmol) of the white crystal of 2-hydroxy6-hydroxycarbonyl-3-naphthoic acid hydrazide (formula (II)) was obtained.

Mass spectrum: m/z (-) 245, m/z (+) 247 (MW 246.2)

Decomposition point: 348°C

The infrared absorption spectrum (KBr method) of the resulting 2-hydroxy-6-hydroxycarbonyl-3-naphthoic acid hydrazide is shown in Figure 2.

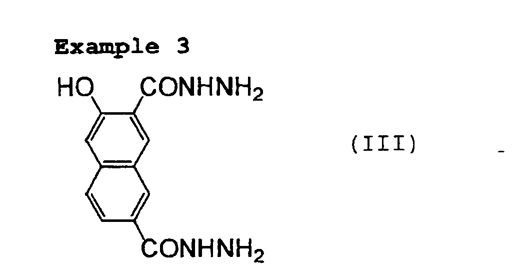

According to the same manner as described in Example 1 with the exception that 21.5 g of 2-hydroxy-3,6-di(n-butoxycarbonyl)naphthalene was used in stead of 2-hydroxy-3-hydroxycarbonyl-6-n-butoxycarbonylnaphthalene, 12.7 g (48.8 mmol) of the white crystal of 2-hydroxy-3,6-bis(hydrazinocarbonyl)naphthalene (formula(III)) was obtained.

Mass spectrum: m/z (-) 259, m/z (+) 260 (MW 260.3)

Melting point: 245°C, Decomposition point: 294°C

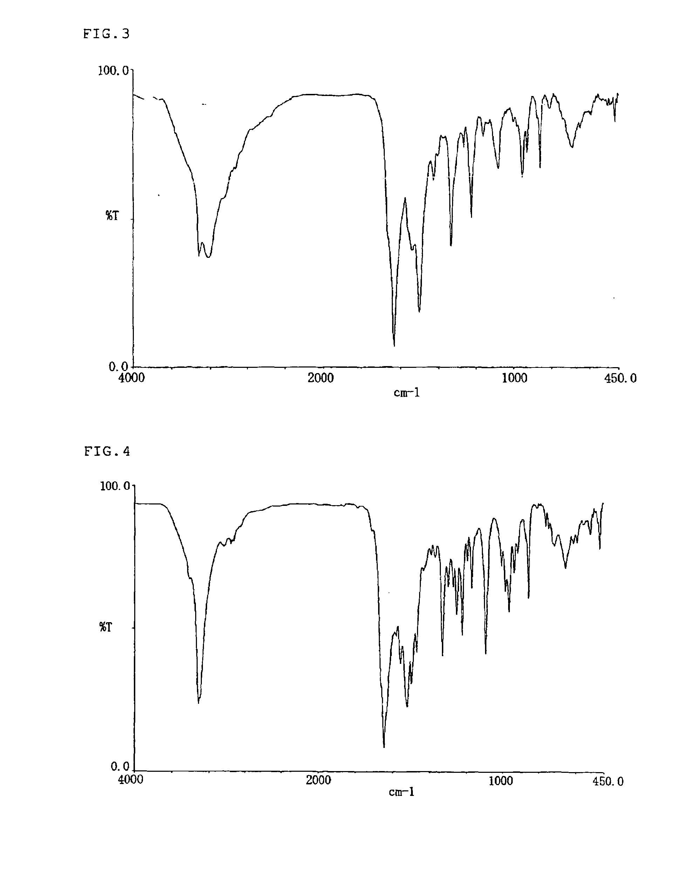

The infrared absorption spectrum (KBr method) of the resulting 2-hydroxy-3,6-bis(hydrazinocarbonyl)naphthalene is shown in Figure 3.

### Example 4

2-Hydroxy-3,6-bis(hydrazinocarbonyl)naphthalene obtained in Example 3 can also be synthesized as follows.

According to the same manner as described in Example 1 with the exception that 18.0 g of 2-hydroxy-3-aminocarbonyl-6-n-butoxycarbonylnaphthalene was used in stead of 2-hydroxy-3-hydroxycarbonyl-6-n-butoxycarbonylnaphthalene, and 100 g of 2-ethylhexylalcohol was used in stead of n-butanol, 14.0 g (53.7 mmol) of the white crystal -- of 2-hydroxy-3,6-bis(hydrazinocarbonyl)naphthalene (formula(III)) was obtained.

Mass spectrum: m/z (-) 259, m/z (+) 261(MW 260.3)

Melting point: 248°C, Decomposition point: 294°C

### Example 5

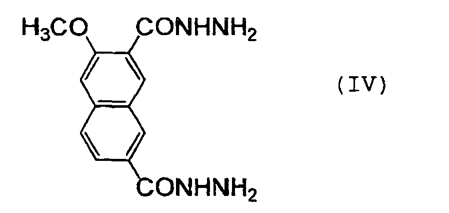

According to the same manner as described in Example 1 with the exception that 18.8 g of 2-methoxy-3-aminocarbonyl-6-n-butoxycarbonylnaphthalene was used in stead of 2-hydroxy-3-hydroxycarbonyl-6-n-butoxycarbonylnaphthalene, and 100 g of 2-ethylhexylalcohol was used in stead of n-butanol, 13.9 g (50.6 mmol) of the white crystal of 2-methoxy-3,6-bis(hydrazinocarbonyl)naphthalene (formula(IV)) was obtained.

Mass spectrum: m/z (+) 275 (MW 274.3)

Melting point: 230°C, Decomposition point: 256°C

The infrared absorption spectrum (KBr method) of the resulting 2-methoxy-3,6-bis(hydrazinocarbonyl)naphthalene is shown in Figure 4.

### Example 6

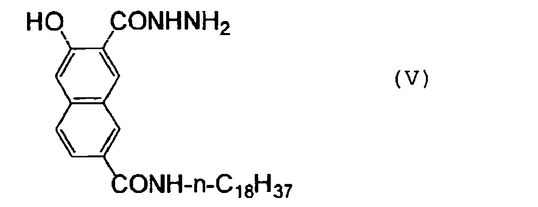

According to the same manner as described in Example 1 with the exception that 62.2 g of 2-hydroxy-3-methoxycarbonyl-6-stearylaminocarbonylnaphthalene was used in stead of 2-hydroxy-3-hydroxycarbonyl-6-n-butoxycarbonylnaphthalene, and 200 g of n-butanol was used in stead of 100 g of n-butanol, 56.9 g (112.5 mmol) of the white crystal of 2-hydroxy-6-stearylaminocarbonyl-3-naphthoic acid hydrazide (formula(V)) was obtained.

Mass spectrum: m/z (-) 497, m/z (+) 499 (MW 497.7)

Melting point: 216°C, Decomposition point: 359°C

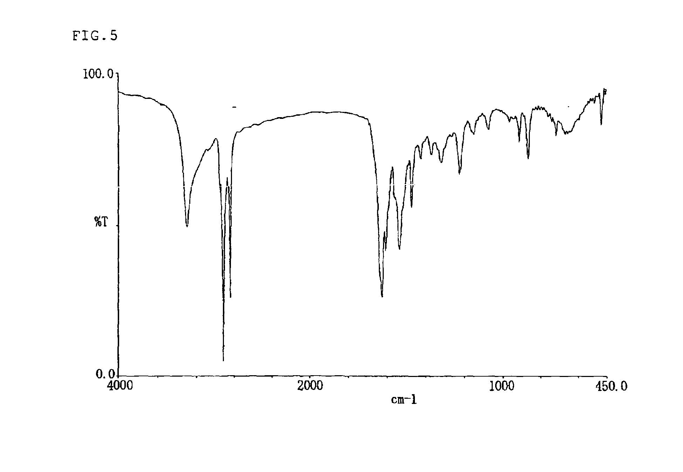

The infrared absorption spectrum (KBr method) of the resulting 2-hydroxy-6-stearylaminocarbonyl-3-naphthoic acid hydrazide is shown in Figure 5.

## Claims

1. A hydroxynaphthalenedicarboxylic acid hydrazide or a derivative thereof represented by formula (1): 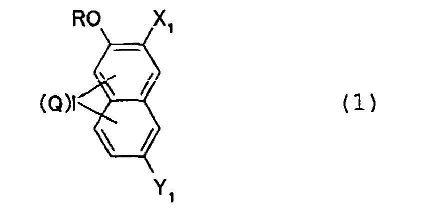 wherein X₁ is a group selected from the group consisting of carboxyl group, a group represented by formula (2) and a group represented by formula (3):
-CO-NH-Z (2)
-CO-NHNH₂ (3)
wherein Z is a group selected from the group consisting of a an optionally branched, optionally substituted, saturated or unsaturated aliphatic group having 1-20 carbon atoms, an optionally substituted aromatic group and an optionally substituted heterocyclic group having conjugated double bonds;
Y₁ is a group selected from the group consisting of carboxyl group, carbamoyl group, a group represented by formula (2), a group represented by formula (3), and a group represented by formula (4):
-CO-O-A (4)
wherein A is alkyl group having 1-6 carbon atoms;
provided that at least one of X₁ and Y₁ in formula (1) is a group represented by formula (3);
R is selected from the group consisting of hydrogen atom, an optionally branched alkyl group having 1-20 carbon atoms which may be optionally substituted with hydroxyl group and/or halogen atom and aralkyl group having 7-11 carbon atoms;
Q is selected from the group consisting of alkyl group having 1-6 carbon atoms, alkoxy group having 1-6 carbon atoms, halogen atom, nitro group and hydroxyl group;
1 represents an integer of 0-5;
provided that when 1 is from 2 to 5, the Qs may be the same or different.

2. A process for preparing the hydroxynaphthalenedicarboxylic acid hydrazide or derivative thereof according to claim 1, which comprises: reacting a hydroxynaphthalenedicarboxylic acid derivative represented by formula (5) with one or more hydrazine compound selected from the group consisting of hydrazine monohydrate, hydrazine sulfate, dihydrazine sulfate, hydrazine monohydrochloride, hydrazine dihydrochloride and hydrazine hydrobromide at a temperature of 20-110°C: 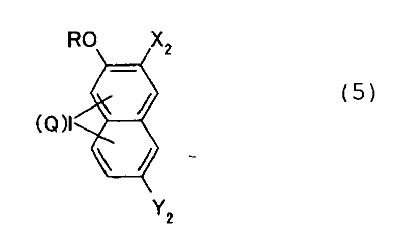 wherein X₂ and Y₂ are independently selected from the group consisting of a group represented by formula (2), a group represented by formula (3), a group represented by formula (4), carbamoyl group and carboxyl group;
provided that at least one of X₂ and Y₂ is a group represented by formula (4) or carbamoyl group;
R, Q and 1 are the same as defined in claim 1.

3. The process according to claim 2, wherein said hydrazine compound is hydrazine monohydrate.
